(19) Europäisches Patentamt
European Patent Office
Office européen des brevets

(11) EP 3 429 579 B1

(12) FASCICULE DE BREVET EUROPEEN

(45) Date de publication et mention de la délivrance du brevet:
**02.09.2020 Bulletin 2020/36**

(21) Numéro de dépôt: **17714859.0**

(22) Date de dépôt: **14.03.2017**

(51) Int Cl.:
***A61K 31/352*** *(2006.01)* ***A61K 31/7052*** *(2006.01)*
***A61P 31/00*** *(2006.01)* ***A61P 31/04*** *(2006.01)*

(86) Numéro de dépôt international:
**PCT/FR2017/050581**

(87) Numéro de publication internationale:
**WO 2017/158282 (21.09.2017 Gazette 2017/38)**

# (54) TULATHROMYCINE POTENTIALISEE

VERBESSERTES TULATHROMYCIN

ENHANCED TULATHROMYCIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.03.2016 FR 1652110**

(43) Date de publication de la demande:
**23.01.2019 Bulletin 2019/04**

(73) Titulaire: **Septeos**
**75017 Paris (FR)**

(72) Inventeur: **TESSE, Nicolas**
**92420 Vaucresson (FR)**

(74) Mandataire: **Regimbeau**
**20, rue de Chazelles**
**75847 Paris Cedex 17 (FR)**

(56) Documents cités:
**WO-A1-98/56802** **WO-A1-2012/001089**
**US-A1- 2014 039 045**

- **JEDLICKOVÁ Z ET AL: "Antibacterial properties of the Vietnamese cajeput oil and ocimum oil in combination with antibacterial agents", JOURNAL OF HYGIENE, EPIDEMIOLOGY, MICROBIOLOGY AND IMMUNOLOGY, AVICENUM, PRAGUE, CS, vol. 36, no. 3, 1 janvier 1992 (1992-01-01), pages 303-309, XP009096925, ISSN: 0022-1732**
- **HENDRY E R ET AL: "Antimicrobial efficacy of eucalyptus oil and 1,8-cineole alone and in combination with chlorhexidine digluconate against microorganisms grown in planktonic and biofilm cultures", JOURNAL OF ANTIMICROBIAL CHEMOTHERAPY, OXFORD UNIVERSITY PRESS, GB , vol. 64, no. 6 1 décembre 2009 (2009-12-01), pages 1219-1225, XP002715614, ISSN: 0305-7453, DOI: 10.1093/JAC/DKP362 Extrait de l'Internet: URL:http://jac.oxfordjournals.org/content/ 64/6/1219 [extrait le 2009-10-16]**
- **Ioana Marinas ET AL: "Rosmarinus officinalis essential oil as antibiotic potentiator against Staphylococcus aureus", Biointerface Research in Applied Chemistry, 15 février 2012 (2012-02-15), pages 271-276, XP055086305, Extrait de l'Internet: URL:http://biointerfaceresearch.com/wp-con tent/uploads/downloads/2012/02/35.BRIAC.Gr umezescu.pdf**
- **MULYANINGSIH S ET AL: "Synergistic properties of the terpenoids aromadendrene and 1,8-cineole from the essential oil of Eucalyptus globulus against antibiotic-susceptible and antibiotic-resistant pathogens", PHYTOMEDICINE, GUSTAV FISCHER VERLAG, STUTTGART, DE, vol. 17, no. 13, 1 novembre 2010 (2010-11-01), pages 1061-1066, XP027417669, ISSN: 0944-7113 [extrait le 2010-08-19]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet les composés de formule (I), utilisés à une dose où ils ne présentent plus de propriétés antimicrobiennes, pour leur utilisation en tant qu'agent destinés à la potentialisation de la tulathromycine avec laquelle ils sont co-administrés. En particulier, la combinaison « potentialisateur(s) + tulathromycine » a pour but de prévenir et/ou traiter les infections bactériennes et fongiques chez l'animal.

L'invention a également pour objet un procédé pour potentialiser la tulathromycine dans lequel on co-administre avec la tulathromycine un composé (ou plusieurs) de formule (I). Dans ce procédé, on utilise le composé de formule (I) à une dose où il est inactif seul.

## OBJET DE L'INVENTION

### Background

L'invention a pour objet d'apporter des solutions aux problématiques liées à la baisse ou la perte d'activité des antibiotiques durant leur période d'utilisation commerciale et médicale.

Après l'arrivée des antimicrobiens dans les années 1940 il est rapidement apparu que les microbes (bactéries et champignons) avaient la capacité de s'adapter aux antimicrobiens utilisés. L'efficacité de ceux-ci diminue au fil du temps et de leur utilisation. 2 stratégies existent pour lutter contre la résistance, la découverte de nouvelles molécules antimicrobiennes d'une part, et d'autre part l'association avec des molécules destinées à bloquer sélectivement les mécanismes de résistance.

Depuis une vingtaine d'années, il est observé une baisse du nombre de nouvelles molécules antimicrobiennes accédant au marché, ce qui a entrainé l'augmentation majeure de la prévalence mondiale de microbes résistants. Il s'ensuit alors une situation complexe pour les patients qui sont plus difficilement soignés de leurs infections microbiennes.

La Demanderesse a déjà démontré, dans la demande PCT/EP2015/071093, que les composés de formule (I) étaient capables de potentialiser des antibiotiques. Dans cette demande, il a été démontré que les bons résultats *in vitro* étaient confirmés *in vivo.* La demande PCT/EP2015/071093 ne cite pas la tulathromycine.

La tulathromycine est un antibiotique de la famille des macrolides (voir par exemple WO 98/56802 ou WO 2012/001089). Son nom chimique (nomenclature IUPAC) est le (2R,3S,4R,5R,8R,10R,11R,12S,13S,14R)-13-[[2,6-dideoxy-3-C-methyl-3-O-methyl-4-C-[(propylamino)methyl]-α-L-ribo-hexopyrano-syl]oxy]-2-ethyl-3,4,10-trihydroxy-3,5,8,10,12,14-hexamethyl-11-[[3,4,6-trideoxy-3-(dimethylamino)-β-D-xylo-hexopyranosyl]-oxy]-1-oxa-6-azacyclopentadecan-15-one. Elle est commercialisée par Zoetis sous la marque Draxxin® pour le traitement du bétail et des cochons ou autres animaux domestiqués.

Chez les bovins, la tulathromycine est notamment indiquée pour le traitement, la metaphylaxie et la prévention des pathologies respiratoires bovines associées à *Mannheima haemolytica, Pasteurella multocida, Histophilus somni* et *Mycoplasma bovis* sensibles à la tulathromycine. Elle est également indiquée pour le traitement et la metaphylaxie de la kératoconjonctivie infectieuse bovine associée à *Moraxella bovis* sensible à la tulathromycine.

Chez les porcins, la tulathromycine est notamment indiquée pour le traitement, la metaphylaxie et la prévention des pathologies respiratoires porcines associées à *Actinobacillus pleuropneumoniae, Pasteurella multocida, Mycoplasma hyopneumoniae* et *Haemophilus parasuis* sensibles à la tulathromycine.

### Descriptif

L'invention décrit l'utilisation de composés de formules (I) pour potentialiser la tulathromycine. De façon inattendue, ces composés ont démontré leur capacité de potentialisation de l'effet de la tulathromycine à des doses faibles (de 0,01 à 100 mg/l) très éloignées de celles où ils peuvent, seuls, présenter des propriétés antimicrobiennes. En conséquence, la tulathromycine co-administrée avec les composés de formules (I) présente, du fait de la potentialisation, une activité supérieure à celle usuellement observée.

Il est important de rappeler que toute molécule présente, dans l'absolu, des propriétés antimicrobiennes. Les propriétés antimicrobiennes d'une molécule doivent donc s'évaluer en regard de la concentration minimale qui inhibe la bactérie.

L'invention se base sur la découverte surprenante que des composés, qui présentent des propriétés antimicrobiennes à des doses très élevées (niveau incompatible avec un usage en médecine pour cet effet) et dont les concentrations minimale inhibitrice (CMI) sont supérieures à 5000 mg/L, avantageusement supérieures à 10 000 mg/L, présentent des effets de potentialisation à des doses faibles (0,01 à 100 mg/l). A ces doses, il devient envisageable d'utiliser les composés (I) chez l'animal.

De façon encore aussi surprenante, il a été constaté que l'effet de potentialisation est meilleur à dose faible du composé (I), et que l'effet (pour les composés présentant des propriétés antimicrobiennes à dose très élevées)

augmente lorsque l'on s'éloigne de la dose à laquelle le composé (I) présente seul des propriétés antimicrobiennes.

Par exemple, il a été constaté de façon inattendue que le cinéole, qui présente des propriétés antimicrobiennes à une concentration de l'ordre du g/L (CMI de 25 000mg/l à 50 000 mg/l soit 2,5 à 5%, selon les souches), présente des effets de potentialisation des antibiotiques à ces concentrations proches de la CMI (1 à 3 dilutions).

Lorsque l'on diminue sa concentration en dessous de la CMI, le cinéole ne potentialise plus les antibiotiques (voire exemples 2 et 6 de la demande PCT/EP2015/071093). Puis, de manière très surprenante, on a constaté qu'en diminuant encore significativement la concentration en cinéole, de 10 à 50 fois moins que la CMI, le cinéole présente à nouveau des effets de potentialisation sur un très grand nombre d'antibiotiques sur un très grand nombre de souches, cette fois à des doses compatibles avec l'usage médical ou vétérinaire.

Un grand avantage est que les composés (I) utilisés à ces doses éloignées de leur seuil d'efficacité, sont le plus souvent utilisables chez l'animal sans contraintes de toxicité du fait de l'absence de toxicité ou à des niveaux acceptables de toxicité. Cela ne serait pas le cas si l'on souhaitait les utiliser aux doses élevées où ils présentent des propriétés antimicrobiennes voire aux doses proche de leur CMI ou ils pourraient potentialiser les antimicrobiens.

De façon tout aussi surprenante l'effet de potentialisation observé avec le composé selon l'invention n'est pas spécifique d'un mécanisme de résistance particulier mais s'observe sur diverses souches, qu'elles aient, ou n'aient pas, développé un ou plusieurs mécanismes de résistance distincts.

**ART ANTERIEUR**

L'art antérieur décrit dans de nombreuses publications les propriétés antimicrobiennes de certains composés (I). Ces publications n'anticipent pas l'effet de potentialisation des composés (I) à des doses éloignées de leur seuil d'efficacité.

L'art antérieur est conséquent sur les composés (I) car ils appartiennent à des classes chimiques très étudiées dans de nombreux domaines. Les composés (I) dans le cadre de la présente invention présentent les caractéristiques suivantes qui permettent de les écarter de l'art antérieur et qui permettent d'envisager leur usage comme potentialisateur d'antimicrobiens administrés à l'homme et à l'animal:

A/ Ils sont utilisés à des doses faibles (0,01 à 100 mg/l), doses éloignées de leur seuil de propriétés antimicrobienne, leur effet de potentialisation n'est pas spécifique de mécanismes d'action ou de résistance particuliers
B/ ils ont été validés par des méthodes de screening adaptée à leurs caractéristiques physico-chimiques
C/ ils présentent, aux doses ou ils potentialisent, un ratio effet/toxicité qui permet un usage sécurisé chez l'homme ou l'animal
D/ Ils constituent une (des) entité(s) chimique(s) isolée(s) dont les caractéristiques sont reproductibles de façon constante à l'infini car leur méthode d'obtention (synthèse, hémi-synthèse, extraction) le permet.
E/ Ils ne possèdent pas de toxicité particulière qui interdirait leur usage même à dose faible (génotoxicité, cardio-toxicité,...)

<u>A/ Concernant les niveaux d'activités mesurés dans l'art antérieur</u>

Dans l'art antérieur, la concentration utilisée pour observer une activité antimicrobienne n'est pas compatible avec une utilisation future chez l'homme ou l'animal, en particulier lors d'une application systémique. Dans la majeure partie des publications scientifiques, l'usage médical est envisagé alors que les concentrations efficaces mesurées (de l'ordre de plusieurs mg/ml) sont incompatibles avec cet usage. Les effets antimicrobiens mesurés l'ont très souvent été pour des teneurs en huile essentielle, ou de son composé actif, de l'ordre de plusieurs mg/ml. Or, une telle concentration n'est pas adaptée pour une utilisation future chez l'homme ou l'animal, en particulier par voie systémique. 1 mg/ml correspond à 1 g/l ou 1g/kg ou encore 0,1%. Si la CMI était de 1 mg/ml, il faudrait, en fonction des paramètres de pharmacocinétique, administrer au moins 1 g/kg/j de poids vif. Par exemple, la dose effective pour une vache devrait être au moins 500 g/j et au moins 60 g/j pour l'homme (ceci correspond à la dose minimale car on suppose ici que le produit est totalement absorbé et distribué dans l'organisme). Ces doses bien trop importantes ne sont pas envisageables pour une utilisation sécurisée en thérapeutique.

Par exemple on peut citer deux publications qui citent l'effet antibactérien des terpénoïdes et leur éventuel capacité de potentialisation des antimicrobiens :

- Biointerfacae Vol 2, Issue 1, 2012, 271-276 : Marinas et al : Rosmarinus officinalis essential oil as antibiotic potentiator against Staphylococcus aureus.
  Si cette publication semble proche de l'invention il est important de noter la dose envisagée (p274) d'eucalyptol qui, à 25 μL/mL (soit 25 ml /L soit 25 g/l) se situe très loin de la dose administrable à l'homme ou l'animal. Le test de synergie envisagée est réalisé avec la solution de stockage (50% de cinéole) soit 50 g/L, supérieure à la CMI du

cinéole dans la publication, dosage incompatible pour une utilisation en médecine.

- Journal of Antimicrobial Chemotherapy (2009) 64, 1219-1225 : Hendry et al : Antimicrobial efficacy of eucalyptus oil and 1,8-cineole alone and in combination with chlorhexidine digluconate against microorganisms grown in planktonic and biofilm cultures.
  Là encore il est envisagé la synergie avec un produit qui présente des propriétés antimicrobiennes. La concentration de cinéole envisagée est de 4 g/L, dose très proche de la CMI du cinéole dans la publication (8-64 g/L) mais trop importante pour être envisageable dans une administration humaine ou animale. Clairement l'invention se différencie de ces deux publications car dans celles-ci les doses envisagées sont très élevées tandis que la concentration envisagée se situe à proximité de la CMI.

B/ Concernant l'inadaptation des méthodes décrites dans l'art antérieur

Les auteurs ayant travaillé selon des approches différentes (produits actifs seuls, produits naturels,...) sur les familles chimiques comprenant les composés (I) ont en général utilisé les méthodes standard de mesure d'effet antibactérien sans les adapter à la nature hydrophobe et volatile des terpénoïdes et phénylpropanoïdes.

Par exemple, WO 99/66796 (Wisconsin Alumni Research Foudation) décrit une méthode pour sensibiliser des cellules microbiennes vis-à-vis de composés antibactériens comprenant une étape de mise en contact avec un composé antibactérien et un sesquiterpenoïde, pour améliorer l'effet du composé antibactérien.

Dans cette demande, les CMI ont été déterminées avec la méthode de diffusion sur gélose, inadaptée à la nature volatile et hydrophobe des composés (I) et des familles apparentés. Cette méthode consiste à déposer des disques de papier imprégnés de quantités connues de composés à tester sur une gélose ensemencée avec la bactérie à étudier. Il s'établit sur la gélose un gradient de concentration du composé autour de chaque disque ; après 18 heures on mesure le diamètre du halo d'inhibition. Cette méthode n'est toutefois pas fiable pour les composés hydrophobes qui, en raison de tensions de surface et d'angles de contacts très différents sur les surfaces hydrophiles, interfèrent avec la formation du gradient de concentration dans la gélose. Dans certaines zones, la concentration en composés à tester est bien supérieure à la concentration théorique. Ainsi, les tests ne peuvent pas être quantitatifs, lorsqu'ils peuvent être qualitatifs. En outre, on note une dilution du composé hydrophobe à tester dans l'éthanol, sans toutefois corriger le résultat alors que l'éthanol est un composé antibactérien et volatil.

A noter que cette demande enseigne qu'aucun effet n'est obtenu avec des terpènes autres que les sesquiterpènes.

C/ Concernant la toxicité des composés de l'art antérieur

A propos des composés et compositions naturelles, il y a une confusion entre l'origine naturelle et l'absence de toxicité. Les huiles essentielles (et leurs dérivés) sont habituellement décrites comme étant peu toxiques, ce qui est souvent vrai dans des applications alimentaires ou en parfumerie mais erroné dans le cadre d'une administration thérapeutique.

A propos des composés chimiques isolés, la confusion existe aussi et repose sur l'origine naturelle (extraction) des composés.

Par exemple WO2006/120567 (Advanced Scientific developments) décrit des compositions pharmaceutiques comprenant au moins une substance thérapeutique active et décrite comme non toxique, choisie parmi carveol, thymol, eugenol, borneol, carvacrol, alpha-ionone, beta-ionone, et leurs isomères, dérivés et mélanges, et comprenant, à titre de deuxième substance thérapeutique active un antibiotique. Le carveol, thymol, eugenol, borneol, carvacrol, alpha-ionone, ou beta-ionone, utilisés seuls, présentent une activité antibactérienne et nombre d'entre eux posent cependant également des problèmes de toxicité, ignorés dans cette demande.

Par exemple, le carvacrol présente les données de toxicité suivantes: la DL 50 (souris, intraveineuse) est de 80 mg/kg tandis que la dose létale la plus faible en voie orale est de 100 mg/kg, chez deux espèces mammifères (chat et rat). Ces données sont à mettre en regard de la dose de 0,3 mg/ml (soit 300 mg/kg), envisagés dans le document.

D/ Concernant la variabilité chimique des composés décrits dans l'art antérieur

L'utilisation d'huile essentielle est problématique, à l'échelle industrielle, en termes de qualité et de reproductibilité étant donné que la composition d'une huile essentielle varie d'un lot à l'autre.

Par exemple DE 196 31 037 (Boehringer) décrit l'utilisation de l'huile essentielle d'arbre à thé pour potentialiser l'effet d'antibiotiques sur les souches Staphylococcus aureus. Le composant principal l'huile essentielle d'arbre à thé est le terpinen-1-ol.

Cette variabilité a notamment trois conséquences qui limitent industrialisation en vue d'une application chez l'homme ou l'animal:

- il est difficile d'assurer la constance de l'effet thérapeutique
- Il est difficile d'assurer la faible toxicité des produits
- le coût relatif à l'approvisionnement et au management de la qualité et de la reproductibilité des matières est important.

Le tableau suivant récapitule l'enseignement de ces arts antérieurs :

Tableau 1

| | A/ Produits actifs à dose envisagée | B/ Méthode de screening incompatible avec la nature chimique des composés | C/ Toxicité à la dose envisagée | D/ Problème de variabilité chimique | Dose envisagée |
|---|---|---|---|---|---|
| Marinas et al | Oui | Oui | Oui | Non | 15 g/L soit 15 000 mg/l |
| Hendry et al | Oui | Non | Oui | Non | 4 g/L soit 4 000 mg/l |
| WO2009/043987 Aromatechnologies | Oui | Oui | Oui | Oui | 0,1 à 0,4 % soit : 1 à 4 000 mg/L |
| WO2006/120567 Advanced | Oui | Oui, (pas d'agent dispersant) | Oui | Non | 0,3 mg/ml soit 3 000 mg/l |
| DE 19631037 Boehringer | Oui | Non (test dans le lait) | Oui | Oui | 1 à 2 mg /ml soit 1000 à 2000 mg /l |
| WO 99/66796 Wisconsin Alumni | Oui | Oui | Oui | Non | 1 mM soit 222 mg/L (pour le sesquiterpenoides) |
| Présente Invention | Non | Non | Non | Non | 0,01 à 100 mg/L |

Le travail portant sur des composés (I) à des doses faibles éloignées de celles auxquelles ils présentent une activité antimicrobienne est nouveau et l'art antérieur n'a pas, à notre connaissance, envisagé cet usage.

**DEFINITIONS**

Par « micro-organisme », on entend tout organisme vivant, invisible à l'œil nu en raison de ses faibles dimensions.

Par « organisme », on entend toute entité biologique (être vivant) animale ou végétale capable de naître, de se développer et normalement de se reproduire.

Dans ce brevet la définition de microbe reprend la définition de micro-organisme, limitée au champ médical auquel l'invention se réfère. Ainsi les « microbes » sont les microorganismes vivants potentiellement pathogènes (bactéries, champignons, levure et mycobactéries). Le terme exclue donc les pathogènes inertes comme les virus et les prions.

Par « antimicrobien » on entend tout composé destiné à être administré à l'homme ou l'animal capable de tuer ou d'inhiber la croissance de microbes. Les sels pharmaceutiquement acceptables de ces antimicrobiens sont également inclus dans cette définition. Cela inclut, par exemple, les sels de sodium, de potassium, de calcium, etc. et les sels aminés de procaine, dibenzylamine, éthylendiamine, éthanolamine, méthylglucamine taurine, etc., de même que les sels d'addition acide tels que les hydrochlorures et les acides aminés basiques. Le terme regroupe ainsi les antibiotiques (leurs associations avec les inhibiteurs de mécanismes de résistance), les antifongiques destinés à un usage systémique ou local.

Par « propriétés antimicrobiennes » on entend les propriétés d'une quelconque substance capable de détruire ou d'inhiber la croissance des microbes. Les produits qui présentent des propriétés antimicrobiennes comprennent notamment les antimicrobiens et les biocides.

Par opposition au terme « antimicrobien » qui regroupe les antibactériens, antifongiques destinés à être administrés, le terme « biocide » regroupe les produits ayant des propriétés antimicrobiennes destinés à être appliqués à des systèmes inertes (virus et prions).

Par « propriétés antibactériennes » et « propriétés antifongiques » on entend non seulement les propriétés bactéricides et fongicides caractérisées par la destruction des bactéries et champignons (et levures, mycobactéries), mais également les propriétés bactériostatiques et fongistatiques, caractérisées par l'inhibition de la croissance desdites

bactéries et champignons (et levures, mycobactéries). Les produits qui présentent des propriétés antibactériennes ou antifongiques comprennent notamment les antimicrobiens.

Par « bactérie résistante » aux antibiotiques on entend, au sens de la présente invention, une bactérie résistante à au moins un, notamment au moins deux, en particulier au moins trois, voire au moins quatre, antibiotique(s) ou famille(s) d'antibiotique, classiquement utilisé(s).

Par « bactérie multi-résistante » on entend, au sens de la présente invention une bactérie résistante à plusieurs antibiotiques, en particulier pour lesquels la souche devrait être sensible, ou a priori sensible, tout particulièrement une bactérie qui présente au moins deux résistances non naturelles.

On distingue les « résistances naturelles » des « résistances acquises ». Certains antibiotiques n'ont jamais été efficaces, à des doses non toxiques, contre certaines souches ou espèces bactériennes. Il s'agit d'une résistance naturelle. Lorsque des antibiotiques normalement efficaces ne s'avèrent pas ou peu efficaces vis-à-vis d'une bactérie, cette bactérie a développé une résistance acquise.

Une « infection microbienne » au sens de la présente invention désigne une infection provoquée par une ou plusieurs souches microbiennes et inclut les phases allant de la colonisation de l'hôte aux phases pathologiques. L'expression « infection microbienne » englobe donc tout effet néfaste, signe clinique, symptôme ou toute maladie apparaissant chez l'homme ou l'animal suite à la colonisation par le microbe.

Par "terpenoïde" on entend selon l'invention tout composé comprenant un squelette proche d'un terpène. Un « terpène » désigne un dérivé de l'isoprène qui est obtenu par voie biologique par la condensation d'unités en C5, conduisant par exemple aux monoterpènes, sesquiterpenes. Par « proche » on entend que le squelette est similaire à un terpène ou différent en ce qu'au moins un substituant alkyle, normalement présent, peut être absent ou porté par un autre atome. Le squelette peut en outre être substitué par des radicaux variés tels que des radicaux aliphatiques, saturés ou insaturés, linéaires ou cycliques (alkyles, alcényles, alkylènes), oxy, des aldéhydes, des esters, des alcools, des éthers et leurs équivalents soufrés ou azotés. Le terpénoïde peut avantageusement être d'origine naturelle.

Par "phenylpropanoide" on entend selon l'invention tout composé comprenant un squelette proche d'un phénylpropane. Un « phenylpropane » désigne un dérivé obtenu par synthèse biologique à partir du phénylpropane et conduisant à des dérivés en C6 (aromatique)-C3 (aliphatique) ou en C6 (aromatique)-C1 (aliphatique) et aux lactones correspondantes. Par « proche » on entend que le squelette est similaire à un phénylpropane, en particulier on retrouve le motif phényle, ou différent en ce qu'au moins un substituant alkyle, normalement présent, peut être absent ou porté par un autre atome. Le squelette peut en outre être substitué par des radicaux variés tels que des radicaux aliphatiques, saturés ou insaturés, linéaires ou cycliques (alkyles, alcényles, alkylènes), oxy, des aldéhydes, des esters, des alcools, des éthers et leurs équivalents soufrés ou azotés. Le phénylpropanoïde peut avantageusement être d'origine naturelle.

Le terme « prophylaxie » ou « prévenir une infection » tel qu'utilisé dans la présente demande désigne tout degré de retardement dans le moment d'apparition de signes cliniques ou de symptômes de l'infection, ainsi que tout degré d'inhibition de la sévérité des signes cliniques ou symptômes de l'infection, y compris mais sans limitation à, la prévention totale de ladite infection. Ceci nécessite que l'antimicrobien et le composé selon l'invention soient co-administrés à l'homme ou l'animal susceptible d'être colonisé par une souche microbienne à titre préventif, par exemple suite à un acte de chirurgie, d'implantation d'un dispositif médical, un acte médical intrusif. Cette administration prophylactique peut avoir lieu avant, pendant ou après l'acte susceptible de provoquer une infection (en particulier une infection nosocomiale) dans le but d'empêcher, améliorer, et/ou réduire la sévérité de n'importe quelle infection subséquente.

Le terme « traitement » au sens de la présente demande implique que l'antimicrobien et le composé selon l'invention soient co-administrées à un sujet (humain ou animal) au moment de la colonisation ou après la contamination ou la suspicion de contamination par une souche microbienne susceptible de provoquer une infection telle qu'une infection nosocomiale. Le terme « traitement » ou « traiter une infection » inclut donc :

- tout effet curatif (inhibition de la croissance ou destruction du microbe) obtenu grâce à la co-administration antimicrobien + composé selon l'invention ainsi que l'amélioration des signes cliniques ou des symptômes observés de même que l'amélioration de l'état du sujet.
- le ralentissement, l'interruption, ainsi que l'arrêt de la progression de l'infection. La co-administration antimicrobien-composé selon l'invention peut en effet également permettre de ralentir la progression d'un microbe et/ou empêcher complètement ou partiellement une infection microbienne de s'étendre aux tissus environnants et au-delà.
- l'inhibition, l'atténuation ou la prévention des conséquences néfastes de l'infection telles que les dommages cellulaire ou physiologiques provoqués par les toxines produites par certains microbes au niveau des tissus infectés ou avoisinants.

Le terme « metaphylaxie » tel qu'utilisé dans la présente demande désigne tout traitement préventif et systématique d'animaux présumés sains mais soumis à un risque certain.

Le terme « co-administrés » signifie que la tulathromycine et le composé selon l'invention (ou le mélange de composés selon l'invention) sont administrés sous forme combinée ou juxtaposée au sujet. La combinaison inclut toute

association médicamenteuse, toute composition pharmaceutique, tout kit pharmaceutique, et tout médicament comprenant (i) au moins la tulathromycine et (ii) au moins un composé selon l'invention. Les composés (i) et (ii) peuvent être présents sous la forme d'un mélange ou sous la forme de formulations ou compositions distinctes dans ladite combinaison. La combinaison peut également comprendre plusieurs composés selon l'invention, en particulier 2 ou 3 ou plus composés selon l'invention. La combinaison pourrait également comprendre un ou plusieurs autres antibiotiques. Ces constituants forment une unité fonctionnelle du fait d'une indication commune, qui est la mise en œuvre d'un traitement antimicrobien. Cette thérapie combinée est plus spécifiquement destinée à la prophylaxie et/ou au traitement d'infections et de maladies d'origine microbienne, en particulier des infections nosocomiales.

La co-administration peut être simultanée ou étalée dans le temps.

Le terme « simultané » signifie que la tulathromycine et le composé selon l'invention (ou le mélange de composés selon l'invention) sont administrés en même temps, au même moment, à un sujet. Ces composés peuvent être administrés sous la forme d'un mélange ou, simultanément mais séparément, sous la forme de compositions distinctes.

L'expression « administration séquentielle » signifie que la tulathromycine et le composé selon l'invention (ou le mélange de composés selon l'invention) sont administrés non pas simultanément mais séparément dans le temps, l'un après l'autre.

L'expression « potentialiser » la tulathromycine signifie que l'utilisation d'un composé selon l'invention permet d'obtenir un effet prophylactique, metaphylaxique ou thérapeutique supérieur à l'effet prophylactique, metaphylaxique ou thérapeutique obtenu en utilisant tulathromycine seule. Ceci peut s'exprimer de différentes manières, alternatives ou cumulatives : augmentation de l'effet de la tulathromycine, diminution de la dose de tulathromycine à effet constant de tulathromycine, réduction de la CMI. En outre, la potentialisation permet de réduire, voire annihiler, l'apparition de résistance.

L'expression augmenter l'effet de la tulathromycine signifie : l'élargissement du spectre microbien de l'activité de la tulathromycine, l'augmentation de la vitesse d'action de la tulathromycine, l'amélioration du succès clinique (cure rate) ou de la vitesse de l'obtention du succès clinique (time to cure) de la tulathromycine, à dose constante de tulathromycine.

L'expression « diminuer la quantité de tulathromycine utilisée » signifie que l'utilisation de composé selon l'invention permet d'utiliser une quantité de tulathromycine inférieure à la quantité de tulathromycine normalement nécessaire pour obtenir un effet thérapeutique, metaphylaxique ou prophylactique donné lorsque la tulathromycine est administré seule. La diminution de la quantité de tulathromycine utilisée peut être plus ou moins importante ; elle est de préférence d'au moins 10%, et plus préférablement d'au moins 20 %, encore plus préférablement d'au moins 40%, voire 50% ou plus par rapport à la quantité normalement nécessaire pour obtenir un effet thérapeutique ou prophylactique donné.

« CMI » signifie « concentration minimale inhibitrice », qui est la plus faible concentration de substance à laquelle on n'observe plus de croissance microbienne après 18 à 24h de contact dans des conditions favorables à la croissance microbienne

Les tests de mesure de concentration minimale inhibitrice sont réalisés en milieu solide gélosé selon les normes internationales en vigueur (normes CLSI M7-A9 Jan 12) : dispersion des composés à tester dans une gélose Mueller Hinton. Une adaptation relative à l'hydrophobicité des composés et compositions est cependant nécessaire pour les disperser dans le milieu : dilution des composés dans un solvant. Les composés et compositions incorporés dans la gélose peuvent être au préalable dilués dans un ou plusieurs solvant (Tween® 80 dilué dans l'eau, tween® 80 dilué dans le propylène, DMSO dilué dans l'eau). Le milieu peut être ajusté en cations. Les souches sont déposées sur la surface de la gélose avec un appareil de steers. Dans les exemples, différentes méthodes de dissolution des produits sont testées en parallèle pour contourner les problématiques de coefficient de répartition eau/solvant des molécules (antimicrobien et potentialisateur) tandis que les bactéries poussent dans la seule phase aqueuse. La contrainte technique n'interviendra pas dans les tests *in vivo.* Les mêmes méthodes de dilutions peuvent être mise en œuvre en milieu liquide (microplaques et tubes). La même méthodologie est mise en œuvre avec les champignons.

Les CMI 50 et CMI 90 représentent respectivement les concentrations qui inhibent 50% et 90% en nombre des souches d'un même genre.

Un produit présentant un effet génotoxique est un produit qui montre un effet délétère sur le matériel génétique.

La génotoxicité peut être mesurée par le test de Ames, en particulier selon les directives de la conférence internationale sur l'harmonisation (ICH).

Par « atome d'halogène », on entend, au sens de la présente invention, les atomes de fluor, de chlore, de brome et d'iode.

Par « hétéroatome », on entend, au sens de la présente invention, N, O ou S, avantageusement O.

Par groupement « $(C_1-C_6)$alkyle », on entend, au sens de la présente invention, une chaîne hydrocarbonée monovalente saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par groupement « ($C_2$-$C_6$)alcényle », on entend, au sens de la présente invention, une chaîne hydrocarbonée monovalente, linéaire ou ramifiée, comportant au moins une double liaison et comportant 2 à 6 atomes de carbone. A titre d'exemple, on peut citer les groupes éthényle ou allyle.

Par « ($C_1$-$C_6$)halogénoalkyle », on entend, au sens de la présente invention, un groupe ($C_1$-$C_6$)alkyle, tel que défini ci-dessus, pour lequel un ou plusieurs atomes d'hydrogène ont été remplacés par un atome d'halogène tel que défini ci-dessus. Il peut s'agir en particulier d'un groupe CF3.

Par groupement « ($C_1$-$C_6$)alcoxy », on entend, au sens de la présente invention, un groupe ($C_1$-$C_6$)alkyle tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore tert-butoxy.

Par groupement « ($C_2$-$C_6$)alcénoxy », on entend, au sens de la présente invention, un groupe ($C_2$-$C_6$)alcényle, tel que défini ci-dessus, lié au reste de la molécule par l'intermédiaire d'un atome d'oxygène. A titre d'exemple, on peut citer le groupe - $OCH_2CH=CH_2$.

Par groupement « ($C_1$-$C_6$)alkylène » ou « ($C_1$-$C_6$)alcanediyle », on entend, au sens de la présente invention, une chaîne hydrocarbonée divalente, linéaire ou ramifiée, comprenant 1 à 6 atomes de carbone, comme par exemple, un groupement methylène, éthylène, propylène, butylène, pentylène ou encore hexylène.

Par groupement « ($C_2$-$C_6$)alcénylène » ou « ($C_2$-$C_6$)alcènediyle », on entend, au sens de la présente invention, une chaîne hydrocarbonée divalente, linéaire ou ramifiée, comprenant 2 à 6 atomes de carbone et au moins une double liaison, comme par exemple, un groupement vinylène (éthénylène) ou propénylène.

## DESCRIPTION DE L'INVENTION

L'invention a pour objet la tulathromycine potentialisée par un composé de formule I pour son utilisation dans le traitement, la métaphylaxie et la prévention d'une infection microbienne. Elle a ainsi pour objet une association de la tulathromycine et d'un composé répondant à la formule I suivante :

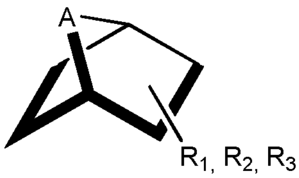

(I)

dans laquelle $R_1$, $R_2$, $R_3$ représentent chacun, indépendamment l'un de l'autre, H, OH un groupe ($C_1$-$C_6$)alkyle, ($C_2$-$C_6$)alcényle, ($C_1$-$C_6$)halogénoalkyle, ($C_1$-$C_6$)alcoxy, ($C_2$-$C_6$)alcénoxy, ($C_1$-$C_6$)halogénoalcoxy, -O-CO-($C_1$-$C_6$)alkyle ;

A représente un hétéroatome ou un groupe R-(Het)-R' ou -(Het)-R où R, R', représentent chacun, indépendamment l'un de l'autre, un groupe ($C_1$-$C_4$)alcanediyle éventuellement substitué par des radicaux alkyles en $C_1$-$C_4$ et Het représente un hétéroatome, A représente avantageusement un groupe -(Het)-R

en un ratio massique, composé de formule (I) : tulathromycine, variant de 8 :1 à 1 :10.

Le ratio massique composé de formule (I) : tulathromycine varie plus avantageusement de 4 :1 à 1 :10, plus avantageusement de 1 :1 à 1 :10, encore plus avantageusement de 1 :1 à 1 :5.

Cela signifie que la dose administrée en composé de formule (I) est du même ordre de grandeur, que celui de la tulathromycine.

Le ratio massique correspond au ratio des doses en mg/kg du composé et de la tulathromycine à administrer à l'homme ou l'animal.

D'une manière surprenante, il a été constaté que l'effet de potentialisation diminue lorsque la dose en composé selon l'invention (ou mélange de composés selon l'invention) augmente. Cet effet de potentialisation peut réapparaître à la CMI du composé seul, mais cela n'est pas l'objet de l'invention.

Dans le cadre de la présente invention, *in vitro,* la concentration en composé inactif, à laquelle on observe un effet de potentialisation, est très éloignée du seuil des propriétés antimicrobiennes (CMI), lorsque des propriétés antimicrobiennes sont observées. Par très éloignée, on entend que la concentration *in vitro* est au moins 10 fois, avantageusement au moins 20 fois, plus avantageusement au moins 50 fois, encore plus avantageusement au moins 100 fois inférieure à la CMI.

En particulier, sur une souche A, pathogène qui induit notamment l'infection microbienne considérée, la concentration en composé (I) répond avantageusement à l'équation suivante :

$$[C] < [CMI] / x$$

Où [C] est la concentration selon l'invention en composé (I) à utiliser sur la souche A
[CMI] est la CMI mesurée pour le composé (I), seul, sur cette souche A
x est supérieur ou égal à 100, avantageusement à 1 000, plus avantageusement x est compris entre 2 000 et 10 000, voire supérieur à 50 000.

*In vitro,* les doses en composé (I) sont inférieures à 100 mg/L, avantageusement inférieures à 64 mg/L, plus avantageusement comprises entre 0,01 et 25 mg/L, encore plus avantageusement comprises entre 1 et 16 mg/L.

Dans les compositions administrées, la concentration, par unité de dose par kilo, en composé de formule (I) est avantageusement inférieure à 100 mg, plus avantageusement inférieure à 64mg.

Les composés (I) peuvent être utilisés à une concentration faible (*in vitro* à des concentrations de l'ordre du $\mu$g/ml) pour potentialiser la tulathromycine, ce qui est tout à fait compatible avec une utilisation future chez l'animal (en particulier si une administration systémique est recherchée).

Cela permet d'envisager des doses chez l'animal inférieures à 64 mg/kg, avantageusement comprises entre 0,01 et 64 mg/kg, plus avantageusement comprises entre 0,1 et 30 mg/kg, encore plus avantageusement comprises entre 0,5 et 10 mg/kg.

Le composé potentialisateur selon l'invention est avantageusement administré à une concentration telle que sa concentration sérique maximale soit inférieure à 250 mg/L, avantageusement inférieure à 150 mg/L, plus avantageusement comprise entre 10 et 150 mg/L après administration.

Bien entendu, à ces concentrations, le composé peut être administré à l'animal, y compris par voie systémique, et ne présente pas d'effets indésirables majeurs, en particulier de carcinogénicité ou de génotoxicité.

De manière générale, les composés de formule (I) ne présentent pas d'effet génotoxique.

Il est intéressant de noter que le cinéole est présent sur la liste des substances autorisées au titre de la règlementation européenne sur les limites maximales de résidus (règlement 37/2010 de la comission du 22 décembre 2010).

La tulathromycine est avantageusement administrée à une dose inférieure à 64 mg/kg, avantageusement comprise entre 0,01 et 64 mg/kg, plus avantageusement comprise entre 0,1 et 30 mg/kg, encore plus avantageusement comprise entre 0,5 et 10 mg/kg.

Pour la tulathromycine dans les cas où elle est usuellement administrée à une dose de 2,5 mg/kg, la dose en composé de formule (I) pourra varier entre 0,75 mg/kg et 2,5 mg/kg, avantageusement entre 0,75 mg/kg et 2 mg/kg voire moins.

Autrement formulée, l'invention a pour objet un procédé pour potentialiser l'activité antimicrobienne de la tulathromycine de façon indépendante du mécanisme de résistance comprenant les étapes suivantes :

a) Choisir un composé de formule (I) qui soit thérapeutiquement inactif (à visée anti-infectieuse) seul à la dose envisagée,
b) Préparer une composition comprenant le composé choisi à l'étape a) avec la tulathromycine

L'invention a également pour objet une méthode pour traiter et/ou prévenir une infection microbienne chez un animal, comprenant la co-administration chez l'animal souffrant ou susceptible de souffrir de ladite infection microbienne de la tulathromycine et d'un composé de formule (I).
Le composé et la tulathromycine sont adaptés pour une administration simultanée, séparée ou étalée dans le temps à l'animal.

D'une manière surprenante, il a été constaté que les composés de formule (I), utilisés à cette faible concentration, sont capables de potentialiser l'activité de la tulathromycine. Ainsi, l'utilisation de ces potentialisateurs permet avantageusement d'utiliser la tulathromycine à une concentration plus faible et/ou, à concentration usuelle tout en ayant une activité supérieure que la tulathromycine seule à la même dose (augmentation de l'intensité de l'effet ou de la cinétique de l'effet).

Concrètement, l'invention permet notamment:

A/ diminuer les doses à effet constant : diminution la quantité nécessaire de tulathromycine pour inhiber/détruire les microbes habituellement sensibles
B/ d'augmenter l'effet à dose constante : augmentation de la capacité de la tulathromycine à inhiber/détruire les germes sensibles (amélioration de la cinétique de l'effet, de l'intensité de l'effet, et élargissement du spectre d'activité de la tulathromycine vers des germes qui étaient inconstamment sensibles ou résistant à la tulathromycine).

Réduire la dose administrée (A/) de tulathromycine présente un intérêt non seulement du point de vue du traitement des infections microbiennes chez l'animal notamment la réduction des effets secondaires, mais également, et ceci n'est pas négligeable, d'un point de vue environnemental (diminution de l'apparition de résistances à la tulathromycine). L'utilisation de la tulathromycine à des doses plus faibles peut aider dans la lutte contre l'apparition de nouveaux mécanismes de résistance. En particulier, la tulathromycine peut être utilisée à une dose réduite, dans laquelle la dose administrée en tulathromycine correspond de 1/50 à 3/4 de la dose nécessaire en tulathromycine en absence de la co-administration d'un composé selon l'invention pour une administration à un animal pour traiter les infections microbiennes. La diminution de la dose de tulathromycine à effet constant permet d'en limiter la toxicité. En application chez l'animal de rente, cela permet de diminuer les temps de latence avant abattage.

Augmenter l'effet de la tulathromycine à dose constante (B/) présente un intérêt clinique certain tant du point de vue quantitatif en améliorant la cinétique d'un effet antimicrobien que qualitatif en rendant possible de traiter un animal souffrant d'une infection microbienne avec la tulathromycine pour laquelle la souche était sensible ou inconstamment sensible en l'absence de potentialisation. L'augmentation de la vitesse de l'effet de la tulathromycine permet de diminuer le temps passé à l'état « infectant » par l'animal, réduisant ainsi l'épidémiologie de la maladie ainsi que l'apparition et la diffusion des résistances.

Grâce à la présence des composés selon l'invention, il est possible d'augmenter la vitesse de bactéricidie de la tulathromycine à dose constante d'antimicrobien. Ainsi, la vitesse d'action de la tulathromycine potentialisée peut être augmentée.

Grâce à la présence des composés selon l'invention, le spectre de la tulathromycine peut être élargi, notamment à dose constante de tulathromycine. Ainsi, la tulathromycine potentialisée par les composés selon l'invention peut être utilisée sur des souches sur lesquelles elle n'est plus sensible en l'absence de potentialisation (notamment en raison de l'apparition de résistances).

Dans un mode de réalisation, le composé de formule (I) est suffisant pour potentialiser la tulathromycine, avec pour conséquence que l'utilisation d'un seul composé de formule (I) est suffisant pour potentialiser la tulathromycine. Cependant, dans certains cas, une utilisation combinée de composés inactifs peut être envisagée.

Dans la formule (I) $R_2$ et $R_3$ représentent chacun H, $R_1$ représente un groupe $(C_1-C_6)$alkyle, et A représente l'hétéroatome O ou un groupe R-O-R' ou -O-R', où R, R', représentent chacun, indépendamment l'un de l'autre, un groupe $(C_1-C_2)$alcanediyle, éventuellement substitué par des radicaux alkyles en $C_1-C_4$.

Avantageusement le composé est de formule (Ia) :

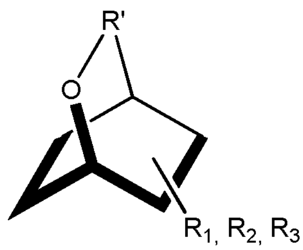

(Ia)

Avec $R_1$, $R_2$, $R_3$ et R' sont tels que définis précédemment, en particulier $R_2$ et $R_3$ représentent chacun H, $R_1$ représente un groupe $(C_1-C_6)$alkyle, R' représente un groupe $(C_1-C_2)$alcanediyle.

Avantageusement le composé de formule (I) est le cinéole.

Dans le cadre de la présente invention, l'infection microbienne est avantageusement une infection induite par un pathogène choisi parmi les genres potentiellement pathogènes suivants: *Acetobacter, Acetobacterium, Acinetobacter, Citrobacter, Enterobacter, Enterococcus, Escherichia, Helicobacter, Histophilus, Haemophilus, Klebsiella, Mycoplasma, Moraxella, Proteus, Providencia, Pseudomonas, Salmonella, Serratia, Staphylococcus, Streptococcus, Actinobacillus, Neisseria, Mannheima, Pasteurella, Candida, Aspergillus, Cryptococcus, Trichosporon, Malassezia,* et *Mycobacterium. En particulier,* un pathogène choisi parmi les genres potentiellement pathogènes suivants: *Mannheima, Pasteurella, Histophilus, Mycoplasma, Moraxella, Actinobacillus, Haemophilus.*

La souche ou espèce bactérienne est avantageusement choisie dans le groupe constitué de: *Acetobacter, Acetobacterium, Acinetobacter, Actinobacillus, Citrobacter, Enterobacter, Enterococcus, Escherichia, Helicobacter, Histophilus, Haemophilus, Klebsiella, Mannheima, Mycoplasma, Moraxella, Pasteurella, Proteus, Providencia, Pseudomonas, Salmonella, Serratia, Staphylococcus,* et *Streptococcus.* Plus particulièrement, la souche ou espèce bactérienne est avantageusement choisie dans le groupe constitué de *Citrobacter freundii, Enterobacter aerogenes, Enterobacter cloacae, Escherichia coli, Klebsiella oxytoca, Klebsiella pneumoniae, Proteus mirabilis, Providencia stuartii, Salmonella sp, Serratia marcescens, Acinetobacter baumannii, Burkholderia cepacia, Pseudomonas aeruginosa, Staphylococcaceae, Staphylococcus aureus, Enterococcus faecium, Enterococcus sp, Mannheima haemolytica, Pasteurella multocida,*

*Histophilus somni, Mycoplasma bovis, Moraxella bovis, Actinobacillus pleuropneumoniae, Pasteurella multocida, Mycoplasma hyopneumoniae, Streptococcus suis et Haemophilus parasuis.* Ainsi, la bactérie peut indifféremment être une bactérie à gram - ou une bactérie à gram +.

La bactérie est plus avantageusement choisie dans le groupe constitué de *Mannheima haemolytica, Pasteurella multocida, Histophilus somni, Mycoplasma bovis, Moraxella bovi,s Actinobacillus pleuropneumoniae, Pasteurella multocida, Mycoplasma hyopneumoniae, Streptococcus suis et Haemophilus parasuis.*

Les composés selon l'invention sont avantageusement administrés par voie systémique. Ils sont ainsi adaptés pour une administration par voie systémique.

Les composés selon l'invention peuvent être utilisés dans toute composition pharmaceutique formulée de façon à faciliter son administration. La composition pharmaceutique peut comprendre tous les excipients pharmaceutiquement acceptables habituellement utilisés tels que des véhicule(s) ou diluant(s).

La composition pharmaceutique peut être administrée par voie orale, entérale, parentérale (intraveineuse, intramusculaire ou sous-cutanée, intraperitoneale), transcutanée (ou transdermique ou percutanée), cutanée, mucosale, (en particulier transmuqueuse-buccale, nasale, ophtalmique, otologique, vaginale, rectale), ou encore les voies intragastrique, intracardiaque, intrapéritonéale, intrapulmonaire ou intratrachéale.

La composition pharmaceutique peut se présenter sous forme sèche, forme sèche à reconstituer au moment de l'utilisation (poudre, lyophilisât, etc.), solide (en particulier cachet, poudre, gélule, pilule, granule, suppositoire, comprimé, et plus précisément comprimé à libération accélérée, comprimé gastrorésistants ou comprimé à libération prolongée), pâteuse (en particulier gel, pommade, crème ou ovule), liquide (en particulier sirop, solution injectable, infusible ou buvable ou collyre), sous forme d'un aérosol (spray, vapeur ou gaz), sous la forme d'un patch, sous forme injectable (dans une solution aqueuse, non aqueuse ou isotonique)

Par ailleurs, la composition pharmaceutique peut être conditionnée pour une administration sous la forme d'une dose unique (monodose) ou multiple (multidose).

La tulathromycine et le ou les composé(s) selon l'invention peuvent être administrés dans une même composition pharmaceutique ou dans des compositions pharmaceutiques distinctes, de manière simultanée, séquentielle ou étalée dans le temps. En cas d'administration séparée, les formes des compositions pharmaceutiques peuvent être similaires ou distinctes ; les voies d'administration pouvant être identiques ou distinctes.

Le schéma d'administration sera adapté par le praticien en fonction des cas. Les voies d'administration et les posologies varient en fonction d'une variété de paramètres, par exemple en fonction de l'état du patient, du type d'infection et de la sévérité de l'infection à traiter.

L'animal est de préférence un mammifère, en particulier l'homme, les animaux de compagnie, les animaux de rente (bovins, ovins, caprins).

Les exemples qui suivent illustrent l'invention.

Mesure de CMI : Les tests de mesure de concentration minimale inhibitrice sont réalisés en milieu solide gélosé selon les normes internationales en vigueur (normes CLSI), selon le protocole défini précédemment. Les composés et compositions incorporés dans la gélose peuvent être au préalable dilués dans un ou plusieurs solvant (Ethanol-, ou le DMSO dilué dans l'eau).

L'antibiotique est la tulathromycine.

Sauf indication contraire, les ratios indiqués dans les tableaux sont des ratios massiques.

## Exemple 1 : Mesure des concentrations minimales inhibitrices

La CMI de la tulathromycine seule, ou combinée avec le cinéole est reportée dans les tableaux 1 à 3 suivants.

Dans ces tableaux, les abréviations suivantes sont utilisées :

ATB = tulathromycine
C = 1,8-cinéole
1/1 : ratio massique ATB/C
X mg = quantité fixe de X mg de cinéole, la quantité de tulathromycine variant en fonction de la CMI
EtOH = éthanol

Tableau 1

| Référence | Nom | ATB EtOH | ATB+C 1/1 EtOH | ATB DMSO | ATB + C 5 mg DMSO |
|---|---|---|---|---|---|
| 13030 | *Actinobacillus pleuropneumoniae* | 0,5 | 0,5 | 0,5 | 0,375 |
| 13032 | *Actinobacillus pleuropneumoniae* | 0,5 | 0,5 | 0,5 | 0,375 |

(suite)

| Référence | Nom | ATB EtOH | ATB+C 1/1 EtOH | ATB DMSO | ATB + C 5 mg DMSO |
|---|---|---|---|---|---|
| 13041 | *Actinobacillus pleuropneumoniae* | 0,5 | 0,5 | 0,5 | 0,375 |
| 13042 | *Actinobacillus pleuropneumoniae* | 0,6 | 0,5 | 0,5 | 0,5 |
| 13043 | *Actinobacillus pleuropneumoniae* | 0,6 | 0,5 | 0,5 | 0,5 |
| 13045 | *Actinobacillus pleuropneumoniae* | 0,6 | 0,5 | 0,5 | 0,5 |
| 13012 | *Actinobacillus pleuropneumoniae* | 2 | 1,5 | 2 | 1,5 |
| 13120 | *E.coli* | 2,7 | 1,5 | 2 | 1,5 |
| 13121 | *E.coli* | 2,7 | 1,5 | 2 | 1,5 |
| 13122 | *E.coli* | 2 | 1,5 | 2 | 1,5 |
| 13123 | *E.coli* | 2 | 1,5 | 2 | 1,5 |
| 13124 | *E.coli* | 2 | 1,1 | 1,5 | 1,1 |
| 13125 | *E.coli* | 1,5 | 0,8 | 1,5 | 1,1 |
| 13126 | *E.coli* | 2 | 1,5 | 2 | 1,5 |
| 13128 | *E.coli* | 2 | 1,5 | 2 | 1,5 |
| 13129 | *E.coli* | 3,6 | 2,7 | 2,7 | 2 |
| 13130 | *E.coli* | 3,6 | 2 | 2 | 1,5 |
| 13131 | *E.coli* | 1,5 | 0,8 | 1,5 | 1,1 |
| 13132 | *E.coli* | 2,7 | 2 | 2 | 1,5 |
| 13133 | *E.coli* | 2 | 1,1 | 1,5 | 1,5 |
| 13134 | *E.coli* | 1,5 | 1,1 | 1,5 | 1,1 |
| 13170 | *E.coli* | 2 | 1,1 | 1,5 | 1,1 |
| 13171 | *E.coli* | 3,6 | 2,7 | 2,7 | 2 |
| 13172 | *E.coli* | 1,5 | 1,1 | 1,5 | 0,8 |
| 13173 | *E.coli* | 1,5 | 1,1 | 1,5 | 1,1 |
| 13174 | *E.coli* | 2 | 1,5 | 2 | 1,5 |
| 13175 | *E.coli* | 3,6 | 2 | 2 | 2 |
| 13092 | *Streptococcus suis* | 1,1 | 0,5 | 1,1 | 0,6 |

Tableau 2

| Référence | Nom | ATB EtOH | ATB + C 1/1 EtOH | ATB + C 1mg EtOH |
|---|---|---|---|---|
| Q102CEA | E coli | 1,42 | 0,8 | 0,8 |
| Q103IEA | E coli | 1,42 | 1,06 | 0,8 |
| Q103JEA | E coli | 1,42 | 1,06 | 0,8 |
| Q96IEB | E coli | 1,42 | 1,42 | 1,07 |
| Q99IEA | E coli | 1,42 | 0,8 | 0,8 |

(suite)

| Référence | Nom | ATB EtOH | ATB + C 1/1 EtOH | ATB + C 1mg EtOH |
|---|---|---|---|---|
| Q103I5 | E coli | 1,42 | 0,8 | 0,8 |
| Q96CEB | E coli | 1,9 | 1,42 | 1,07 |
| Q99JEA | E coli | 1,42 | 0,8 | 0,8 |
| Q97IEA | E coli | 1,42 | 1,06 | 0,8 |
| | E coli | 1,42 | 0,8 | 0,8 |
| | E coli | 1,42 | 0,8 | 0,8 |
| Q103CEA | E coli | 1,42 | 1,06 | 0,8 |
| Q54IE2 | E coli | 0,8 | 0,6 | 0,6 |
| Q96IEB | E coli | 1,42 | 1,06 | 0,8 |
| Q99J1 | E coli | 1,42 | 0,8 | 0,8 |
| Q97JEA | E coli | 1,07 | 0,8 | 0,6 |
| 8152 | Enterococcus | 1 | 0,6 | 0,121 |
| 8153 | Enterococcus | 1,42 | 1,06 | 0,8 |
| 8146 | Staphylococcus | 0,45 | 0,34 | 0,121 |
| 8147 | Staphylococcus | 0,45 | 0,34 | 0,121 |
| 8148 | Staphylococcus | 0,45 | 0,34 | 0,121 |
| 8149 | Staphylococcus | 0,45 | 0,45 | 0,34 |
| 8238 | Staphylococcus | 0,45 | 0,34 | 0,34 |
| 12216 | Staphylococcus | 2,53 | 1,9 | 1,9 |
| 13218 | Staphylococcus | 1,07 | 0,6 | 0,121 |
| 13221 | Staphylococcus | 1,07 | 0,6 | 0,121 |

Tableau 3

| Nom | ATB EtOH | ATB + C 1 mg EtOH | ATB + C 5 mg EtOH |
|---|---|---|---|
| Enterococcus | 0,9 | 0,9 | 0,7 |
| Enterococcus | 1,7 | 1,7 | 1,3 |

On constate que le cinéole est capable de potentialiser la tulathromycine sur un grand nombre de souches.

**Exemple 2 : test de croissance / test de bactéricidie**

Les tests de croissance sont faits en milieu liquide avec une dispersion du booster d'antibiotiques préalable dans un solvant adapté (ici DMSO).

On mesure la cinétique de croissance des bactéries en présence de tulathromycine (à une concentration de 4 fois ou 16 fois sa CMI), de tulathromycine (à une concentration de 4 fois ou 16 fois sa CMI) et de 1,8-cinéole à un ratio massique de 1/1 et de tulathromycine (à une concentration de 4 fois ou 16 fois sa CMI) et de 1 mg de 1,8-cinéole.

CMI de la tulathromycine = 2 mg/L (souche 13120) et 0,5 mg/ (souche 13043),

***1 Test de croissance sur la souche 13120 (genre E. coli)***

Les résultats sont reportés sur la figure 1, dont la légende est :

losange : témoin (bactérie seule dans DMSO)
carré : tulathromycine diluée dans DMSO à une concentration de 4 fois sa CMI
triangle : tulathromycine et cinéole, dilués dans DMSO, la tulathromycine est à une concentration de 4 fois sa CMI, le ratio massique tulathromycine/cinéole est de 1/1
Croix (×) : tulathromycine et cinéole, dilués dans DMSO, la tulathromycine est à une concentration de 4 fois sa CMI, le cinéole à 1mg/L

On constate que la présence de cinéole, à une très faible dose, permet d'augmenter la bactéricidie de la tulathromycine.

***2 Test de croissance sur la souche 13043 (Actinobacillus pleuropneumoniae)***

Les résultats sont reportés sur la figure 2, dont la légende est :

losange : témoin (bactérie seule dans DMSO)
carré : tulathromycine diluée dans DMSO à une concentration de 16 fois sa CMI
triangle : tulathromycine et cinéole, dilués dans DMSO, la tulathromycine est à une concentration de 16 fois sa CMI, le cinéole à 1mg/L
Croix (×) : témoin cinéole, à 1mg/L

On constate que la présence de cinéole, à une très faible dose, permet de décroître significativement le nombre de bactéries alors que la tulathromycine est un antibiotique bactériostatique sur cette souche, lorsqu'elle est utilisée seule.

**Exemple 3 : résistance**

Cent µl d'une culture en bouillon Mueller Hinton de la souche à étudier (inoculum lourd >$10^{10}$ UFC/ml) sont étalés sur une boîte de Mueller Hinton contenant une concentration d'huile égale à 0,5 / 1 / 2 / 4 fois la CMI de la tulathromycine. Après 48h d'incubation on observe la présence ou non de colonies susceptibles d'être des mutants résistants.
On a constaté que l'utilisation de cinéole permettait également de diminuer l'apparition de bactéries résistantes.
Les résultats sont reportés dans les tableaux suivants :

Tableau 4 - Souche 8147 / Staphylococcus - solvant éthanol

| CMI | 4 | 2 | 1 | 0,5 |
|---|---|---|---|---|
| tulathromycine | envahie | envahie | envahie | envahie |
| Tulathromycine + 1mg/L cinéo le | envahie | envahie | envahie | envahie |
| Tulathromycine + 5mg/L cinéole | 0 | 0 | envahie | envahie |

Alors que la tulathromycine seule sur le *S. aureus* entraine l'apparition d'innombrable mutants, l'ajout de cinéole (5mg/L) à la tulathromycine n'entraine plus l'apparition des mutants.

Tableau 5 - Souche 8146 / Staphylococcus - solvant éthanol

| CMI | 4 | 2 | 1 | 0,5 |
|---|---|---|---|---|
| tulathromycine | 0 | envahie | envahie | envahie |
| Tulathromycine + 1mg/L cinéo le | 0 | 2 colonies | envahie | envahie |
| Tulathromycine + 5mg/L cinéole | 0 | 1 colonie | envahie | envahie |

Là encore le cinéole, entraine la diminution d'apparition de mutants.

**Revendications**

1. Association de tulathromycine et d'un composé de formule I pour son utilisation dans le traitement, la métaphylaxie

ou la prévention d'une infection microbienne, le composé répond à la formule I suivante :

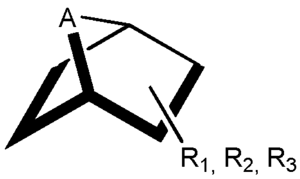
(I)

dans laquelle

$R_1$, $R_2$, $R_3$ représentent chacun, indépendamment l'un de l'autre, H, OH un groupe $(C_1-C_6)$alkyle, $(C_2-C_6)$alcényle, $(C_1-C_6)$halogénoalkyle, $(C_1-C_6)$alcoxy, $(C_2-C_6)$alcénoxy, $(C_1-C_6)$halogénoalcoxy, -O-CO-$(C_1-C_6)$alkyle ;
A représente un hétéroatome ou un groupe -(Het)-R' ou R-(Het)-R' où R, R', représentent chacun, indépendamment l'un de l'autre, un groupe $(C_1-C_4)$alcanediyle éventuellement substitué par des radicaux alkyles en $C_1-C_4$ et Het représente un hétéroatome ;
en un ratio massique, composé de formule (I) : tulathromycine, variant de 8 :1 à 1 :10,
et en ce que l'infection microbienne est induite notamment par un pathogène de souche A et que sur cette souche A, la concentration en composé (I) répond à l'équation suivante :

$$[C] < [CMI] / x$$

Où [C] est la concentration selon l'invention en composé (I) à utiliser sur la souche A
[CMI] est la CMI mesurée pour le composé (I), seul, sur cette souche A
x est supérieur ou égal à 100, avantageusement à 1 000, plus avantageusement x est compris entre 2 000 et 10 000, voire supérieur à 50 000.

2. Association pour son utilisation selon la revendication 1, **caractérisée en ce que** dans la formule (I) $R_2$ et $R_3$ représentent chacun H, $R_1$ représente un groupe $(C_1-C_6)$alkyle, et A représente l'hétéroatome O un groupe R-O-R' ou -O-R' où R, R', représentent chacun, indépendamment l'un de l'autre, un groupe $(C_1-C_2)$alcanediyle éventuellement substitué par des radicaux alkyles en $C_1-C_4$.

3. Association pour son utilisation selon la revendication 1 ou 2, **caractérisée en ce que** le composé de formule (I) est le cinéole.

4. Association pour son utilisation selon l'une des revendications précédentes dans laquelle le ratio massique composé de formule (I) : tulathromycine varie de 4:1 à 1 :10, avantageusement de 1 :1 à 1 :10, plus avantageusement de 1 :1 à 1 :5.

5. Association pour son utilisation selon l'une des revendications précédentes, **caractérisée** en ce la dose en composé (I) est comprise entre 0,01 et 64 mg/kg, avantageusement entre 0,1 et 30 mg/kg.

6. Association pour son utilisation selon la revendication 5, **caractérisée** en ce la dose en composé (I) est comprise entre 0,5 et 10 mg/kg.

7. Association pour son utilisation selon l'une des revendications précédentes, **caractérisée** en ce la dose en tulathromycine est comprise entre 0,01 et 64 mg/kg, avantageusement entre 0,1 et 30 mg/kg.

8. Association pour son utilisation selon la revendication 7, **caractérisée** en ce la dose en tulathromycine est comprise entre 0,5 et 10 mg/kg.

9. Association pour son utilisation selon l'une des revendications précédentes, **caractérisée en ce que** la tulathromycine est administrée à un dose de 2,5 mg/kg, et la dose en composé de formule (I) varie entre 0,75 mg/kg et 2,5 mg/kg, avantageusement entre 0,75 mg/kg et 2 mg/kg.

10. Association pour son utilisation selon l'une des revendications précédentes, **caractérisée en ce que** le composé

de formule (I) est adapté pour une administration par voie systémique.

**Patentansprüche**

1. Mischung aus Tulathromycin und einer Verbindung der Formel I zur Verwendung bei der Behandlung, Metaphylaxie oder Vorbeugung einer mikrobiellen Krankheit, wobei die Verbindung der folgenden Formel I entspricht:

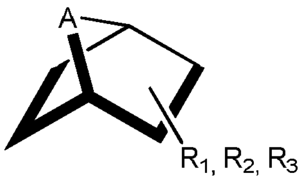

(I)

bei der

$R_1$, $R_2$, $R_3$ jeweils unabhängig voneinander H, OH, eine ($C_1$-$C_6$)-Alkylgruppe, ($C_2$-$C_6$)-Alkenylgruppe, ($C_1$-$C_6$)-Halogenalkylgruppe, ($C_1$-$C_6$)-Alkoxygruppe, ($C_2$-$C_6$)-Alkenoxygruppe, ($C_1$-$C_6$)-Halogenalkoxygruppe, -O-CO-($C_1$-$C_6$)-Alkylgruppe darstellen;
A ein Heteroatom oder eine Gruppe -(Het)-R' oder R-(Het)-R' darstellt, in der R, R', jeweils unabhängig voneinander eine ($C_1$-$C_4$)-Alkandiyl-Gruppe darstellen, die eventuell durch Alkylradikale in $C_1$-$C_4$ substituiert sind und Het ein Heteroatom darstellt;
in einem Massenverhältnis, Verbindung der Formel (I): Tulathromycin, das von 8: 1 bis 1: 10 variiert,
und dass die mikrobielle Infektion insbesondere durch ein Pathogen des Stamms A induziert ist und das in diesem Stamm A die Konzentration in der Verbindung (I) der folgenden Gleichung entspricht:

$$[C] < [MHK] / x$$

in der [C] die Konzentration gemäß der Erfindung in der Verbindung (I) ist, die auf dem Stamm A zu verwenden ist
[CMI] die MHK ist, die für die Verbindung (I) nur auf diesem Stamm A gemessen ist
X größer ist als oder gleich 100, vorteilhaft größer oder gleich 1.000 ist, x noch vorteilhafter zwischen 2.000 und 10.000 inbegriffen bzw. größer als 50.000 ist.

2. Mischung zur Verwendung gemäß Anspruch 1, **dadurch gekennzeichnet, dass** $R_2$ und $R_3$ in der Formel (I) jeweils H darstellen, $R_1$ eine ($C_1$-$C_6$)-Alkylgruppe darstellt und A das Heteroatom O, R-O-R' oder -O-R' in der R, R' darstellt, die jeweils unabhängig voneinander eine ($C_1$-$C_2$)-Alkandiyl-Gruppe darstellen, die eventuell von Alkylradikalen in $C_1$-$C_4$ substituiert sind.

3. Mischung zur Verwendung gemäß Anspruch 1 oder 2, **dadurch gekennzeichnet, dass** die Verbindung der Formel (I) Cineol ist.

4. Mischung zur Verwendung gemäß einem der voranstehenden Ansprüche, bei der das Verbindungs-Massenverhältnis der Formel (I): Tulathromycin von 4: 1 bis 1: 10, vorteilhaft von 1: 1 bis 1: 10, noch vorteilhafter von 1:1 bis 1:5 variiert.

5. Mischung zur Verwendung gemäß einem der voranstehenden Ansprüche, **dadurch gekennzeichnet, dass** die Dosis in Verbindung (I) zwischen 0,01 und 64 mg/kg, vorteilhaft zwischen 0,1 und 30 mg/kg inbegriffen ist.

6. Mischung zur Verwendung gemäß Anspruch 5, **dadurch gekennzeichnet, dass** die Dosis in Verbindung (I) zwischen 0,5 und 10 mg/kg inbegriffen ist.

7. Mischung zur Verwendung gemäß einem der voranstehenden Ansprüche, **dadurch gekennzeichnet, dass** die Tulathromycin-Dosis zwischen 0,01 und 64 mg/kg, vorteilhaft zwischen 0,1 und 30 mg/kg inbegriffen ist.

**8.** Mischung zur Verwendung gemäß Anspruch 7, **dadurch gekennzeichnet, dass** die Tulathromycin-Dosis zwischen 0,5 und 10 mg/kg inbegriffen ist.

**9.** Mischung zur Verwendung gemäß einem der voranstehenden Ansprüche, **dadurch gekennzeichnet, dass** das Tulathromycin in einer Dosis von 2,5 mg/kg verabreicht ist und die Dosis in der Verbindung der Formel (I) zwischen 0,75 mg/kg und 2,5 mg/kg, vorteilhaft zwischen 0,75 mg/kg und 2 mg/kg schwankt.

**10.** Mischung zur Verwendung gemäß einem der voranstehenden Ansprüche, **dadurch gekennzeichnet, dass** die Verbindung der Formel (I) für eine Verabreichung auf systemischem Weg geeignet ist.

## Claims

**1.** Association of tulathromycin and a compound of formula I for the use thereof in the treatment, the metaphylaxis or the prevention of a microbial infection, the compound meets the following formula I:

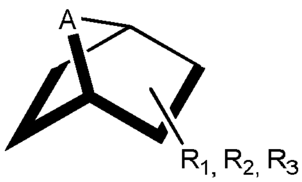

(I)

wherein

$R_1$, $R_2$, $R_3$ each represent, independently of each other, H, OH, a ($C_1$-$C_6$) alkyl, ($C_2$-$C_6$) alkenyl, ($C_1$-$C_6$) halogenoalkyl, ($C_1$-$C_6$) alkoxy, ($C_2$-$C_6$) alkenoxy, ($C_1$-$C_6$) halogenoalkoxy, -O-CO-($C_1$-$C_6$) alkyl group;
A represents a heteroatom or a -(Het)-R' or R-(Het)-R' group where R, R' each represent, independently of each other, a ($C_1$-$C_4$) alkanediyl group optionally substituted by $C_1$-$C_4$ alkyl radicals and Het represents a heteroatom;
in a weight ratio, compound of formula (I) : tulathromycin, varying from 8 :1 to 1 :10, and in that the microbial infection is induced notably by a strain A pathogen and that on this strain A, the concentration of compound (I) meets the following equation:

$$[C] < [MIC] / x$$

Where [C] is the concentration according to the invention of compound (I) to use on the strain A
[MIC] is the measured MIC for the compound (I), alone, on this strain A
x is greater than or equal to 100, advantageously 1,000, more advantageously x is comprised between 2,000 and 10,000, or even greater than 50,000.

**2.** Association for the use thereof according to claim 1, **characterised in that** in the formula (I) $R_2$ and $R_3$ each represent H, $R_1$ represents a ($C_1$-$C_6$) alkyl group, and A represents the heteroatom O, a R-O-R' or -O-R' group where R, R' each represent, independently of each other, a ($C_1$-$C_2$) alkanediyl group optionally substituted by $C_1$-$C_4$ alkyl radicals.

**3.** Association for the use thereof according to claim 1 or 2, **characterised in that** the compound of formula (I) is cineole.

**4.** Association for the use thereof according to one of the preceding claims, wherein the compound of formula (I) : tulathromycin weight ratio varies from 4 :1 to 1 :10, advantageously from 1 :1 to 1 :10, more advantageously from 1 :1 to 1 : 5.

**5.** Association for the use thereof according to one of the preceding claims, **characterised in that** the dose of compound (I) is comprised between 0.01 and 64 mg/kg, advantageously between 0.1 and 30 mg/kg.

**6.** Association for the use thereof according to claim 5, **characterised in that** the dose of compound (I) is comprised between 0.5 and 10 mg/kg.

7. Association for the use thereof according to one of the preceding claims, **characterised in that** the dose of tulathromycin is comprised between 0.01 and 64 mg/kg, advantageously between 0.1 and 30 mg/kg.

8. Association for the use thereof according to claim 7, **characterised in that** the dose of tulathromycin is comprised between 0.5 and 10 mg/kg.

9. Association for the use thereof according to one of the preceding claims, **characterised in that** the tulathromycin is administered at a dose of 2.5 mg/kg, and the dose of compound of formula (I) varies between 0.75 mg/kg and 2.5 mg/kg, advantageously between 0.75 mg/kg and 2 mg/kg.

10. Association for the use thereof according to one of the preceding claims, **characterised in that** the compound of formula (I) is suited for systemic administration.

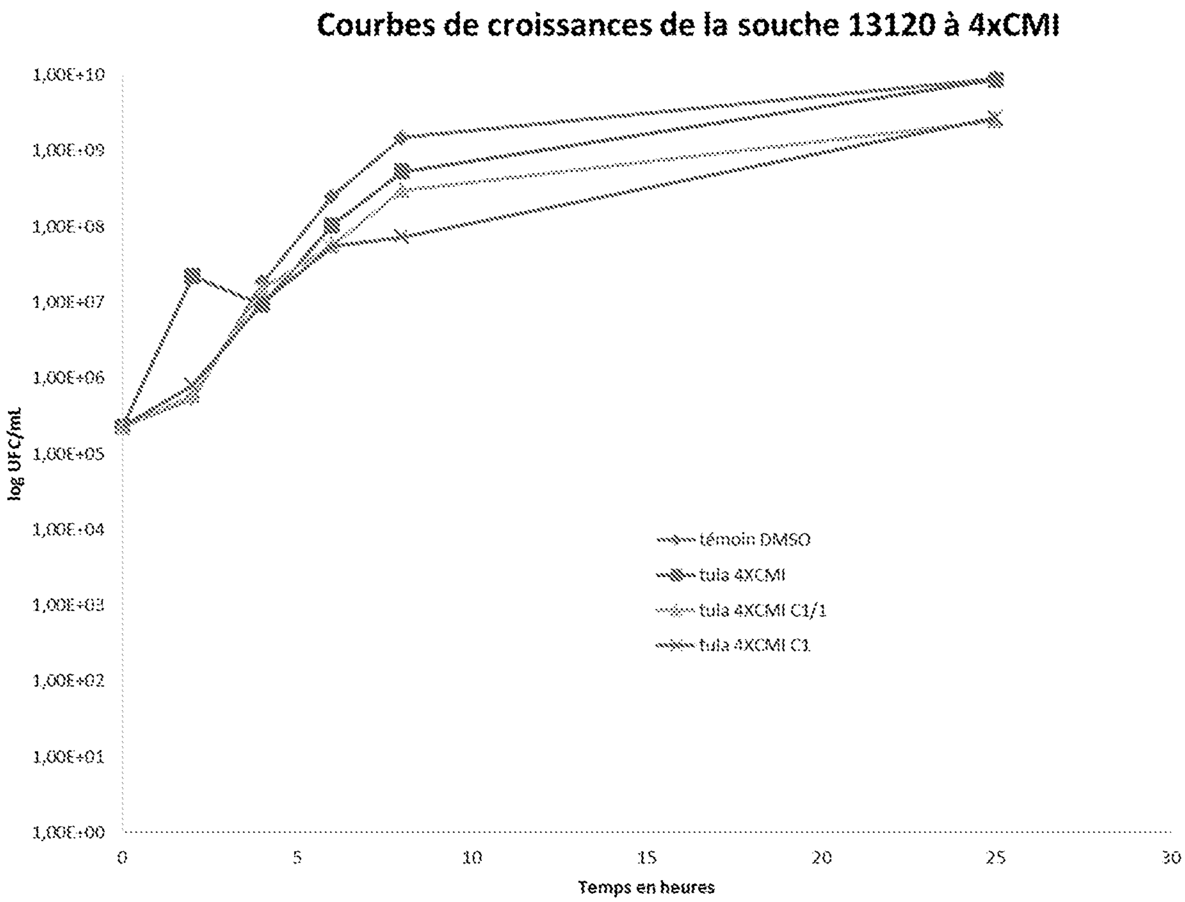
Courbes de croissances de la souche 13120 à 4xCMI
1,00E+10
1,00E+09
1,00E+08
1,00E+07
1,00E+06
1,00E+05
1,00E+04
1,00E+03
1,00E+02
1,00E+01
1,00E+00
log UFC/mL
0
5
10
15
20
25
30
Temps en heures
témoin DMSO
tula 4XCMI
tula 4XCMI C1/1
tula 4XCMI C1

Fig. 1

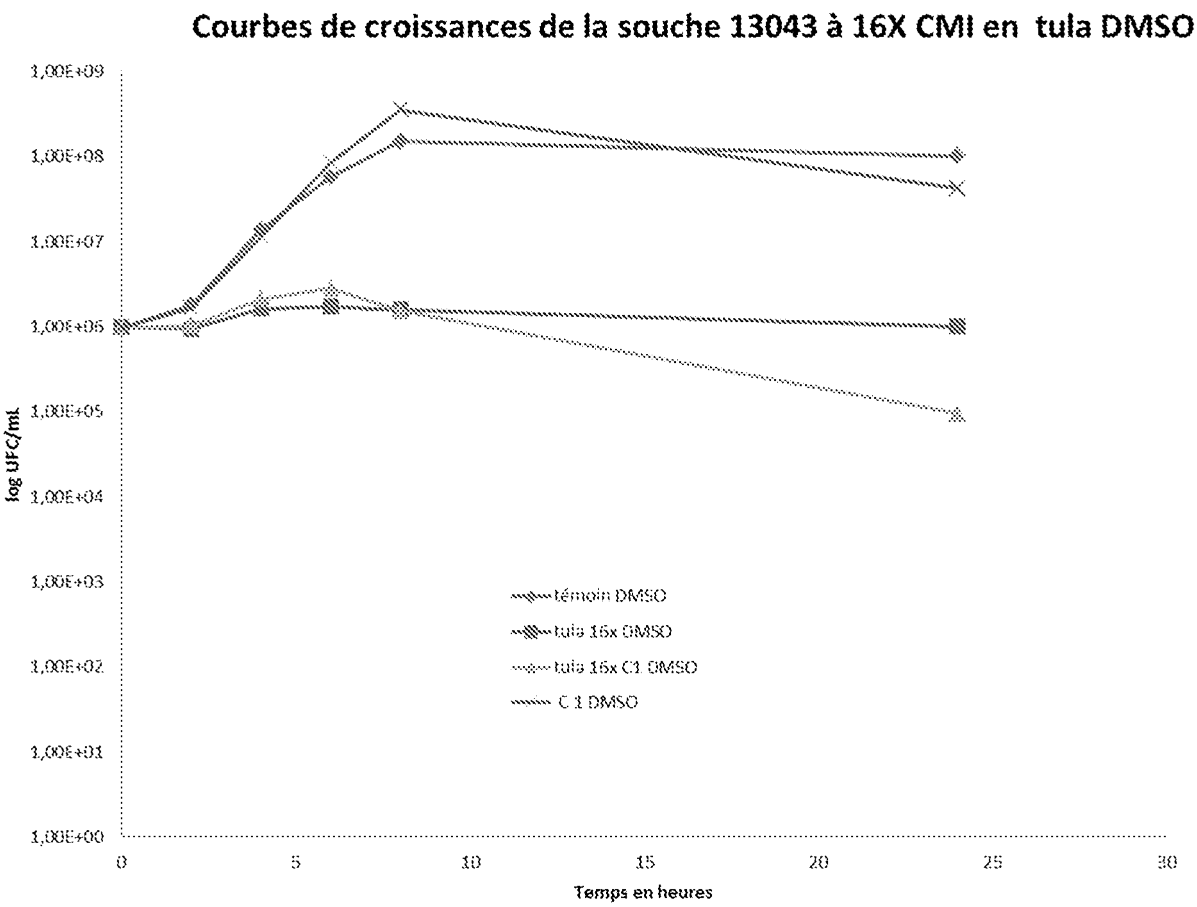
Courbes de croissances de la souche 13043 à 16X CMI en tula DMSO
1,00E+09
1,00E+08
1,00E+07
1,00E+06
1,00E+05
1,00E+04
1,00E+03
1,00E+02
1,00E+01
1,00E+00
log UFC/ml
0
5
10
15
20
25
30
Temps en heures
témoin DMSO
tula 16x DMSO
tula 16x C1 DMSO
C 1 DMSO

Fig. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2015071093 W
- WO 9856802 A
- WO 2012001089 A
- WO 9966796 A
- WO 2006120567 A
- DE 19631037, Boehringer
- WO 2009043987 A


### Littérature non-brevet citée dans la description


- **MARINAS et al.** Rosmarinus officinalis essential oil as antibiotic potentiator against Staphylococcus aureus. *Biointerfacae,* 2012, vol. 2 (1), 271-276
- **HENDRY et al.** Antimicrobial efficacy of eucalyptus oil and 1,8-cineole alone and in combination with chlorhexidine digluconate against microorganisms grown in planktonic and biofilm cultures. *Journal of Antimicrobial Chemotherapy,* 2009, vol. 64, 1219-1225